(19) **Europäisches Patentamt**
**European Patent Office**
**Office européen des brevets**

(11) **EP 3 901 956 A1**

(12) **EUROPEAN PATENT APPLICATION**

(43) Date of publication:
**27.10.2021  Bulletin 2021/43**

(51) Int Cl.:
**G16B 40/30** (2019.01)      **G16H 50/20** (2018.01)
**C12Q 1/68** (2018.01)

(21) Application number: **20170724.7**

(22) Date of filing: **21.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **ETH Zürich**
**8092 Zürich (CH)**

(72) Inventor: **The designation of the inventor has not yet been filed**

(74) Representative: **J A Kemp LLP**
**80 Turmill Street**
**London EC1M 5QU (GB)**

(54) **METHODS OF DETERMINING CORRESPONDENCES BETWEEN BIOLOGICAL PROPERTIES OF CELLS**

(57)    A method of determining a correspondence between a first biological property of a cell and one or more further biological properties of cells is provided. The first biological property and the further biological properties are determined by different analysis techniques and each are contained in a respective one of a plurality of sets of biological properties. The method includes the steps of: converting the plurality of sets of biological properties into corresponding representations in a representation format which is invariant to the technologies used to derive the biological properties; determining, in said representation format, a representation from each of the converted sets of further biological properties which most closely matches the first representation of the first biological property; and reconverting the determined representations from the representation format back to the biological properties associated with the determined representations and thereby determining a correspondence between the first biological property and each of the further biological properties.

1

Fig. 1

**Description**

The present invention relates to methods of determining correspondences between biological properties of cells. In particular, but not exclusively, it relates to a method of determining correspondences between biological properties of cells wherein the biological properties have been determined using different single-cell analysis techniques.

The ability to dissect a tissue into its cellular components to study them individually or to investigate the interplay between the different cell-type fractions is a relatively recent possibility in biological research that has already yielded important insights into the dynamics of various diseases including cancer (Tirosh et al., 2016; Chevrier et al., 2017). Recent advances in single-cell technologies enable molecular profiling of samples with greater granularity at the transcriptomic, proteomic, genomic, and other levels (Rozenblatt-Rosen et al., 2017; Irmisch et al., 2020). Each data modality produces different types and levels of information, but these need to be integrated and related to one another in order to understand the mechanisms at play in the tissue microenvironment and to obtain a more comprehensive molecular understanding of the studied sample. Although technologies capable of measuring two modalities simultaneously are emerging (Stoeckius et al., 2017; Zhu et al., 2020), their scalability and widespread use is still limited.

Furthermore, recent technological advances have led to an increase in the production and availability of single-cell data (both in terms of many modalities and large datasets). In most cases, however, profiling technologies consume the used cells and thus pairwise correspondences between datasets are unavailable or lost. Access to an integrated set of multi-technology measurements would allow, for example, the identification of biologically or clinically meaningful observations through the unification of the perspectives afforded by each technology. However, due to the sheer size of single-cell datasets, scalable approaches that are able to universally match single-cell measurements carried out in one cell to its corresponding sibling in another technology are needed.

Only few approaches have been suggested with similar capabilities. For example "MAGAN" (Amodio and Krishnaswamy, 2018) does not require feature correspondence in principle. However, their results imply that only by including a feature correspondence can accurate matchings be achieved.

Yang and Uhler (2019) propose the use of a latent space from which expression profiles can be generated. However, decoding from the latent space requires generation of expression profiles.

It is thus an object of the present invention to provide a method of determining correspondences between different biological properties of cells, particularly those derived from single-cell analysis techniques.

It is a further object of the present invention to provide a scalable approach to determining such correspondences, in particular in the absence of overlapping features and in the presence of large numbers of observations in two or more technologies.

It is a further object of the present invention to provide an improved matching technique for use in determining correspondences between biological properties of cells. The improvement(s) may, in particular, be in terms of accuracy, computational cost and/or time taken.

It is a further object of the present invention to provide a platform which may be used in an improved clinical decision support system which can make use of combined knowledge of the biological properties of cells derived from different single-cell analysis techniques.

At their broadest, aspects of the present invention provide methods of determining correspondences between biological properties of cells which are determined using different analysis techniques by converting the biological properties into a representation format which is invariant to the technologies used.

A first aspect of the present invention provides a method of determining a correspondence between a first biological property of a cell contained in a first set of biological properties determined by a first analysis technique and a second biological property of a cell contained in a second set of biological properties determined by a second, different, analysis technique, the method including the steps of: converting the first and second sets of biological properties into corresponding representations in a representation format which is invariant to the technologies used to derive the biological properties; determining, in said representation format, a second representation from the converted second set of biological properties which most closely matches a first representation of the first biological property; and re-converting the second representation from the representation format back to the biological property associated with the second representation and thereby determining a correspondence between the first biological property and the second biological property.

A second aspect of the present invention provides a method of determining a correspondence between a first biological property of a cell and a plurality of further biological properties of cells, the first biological property and the further biological properties each being determined by different analysis techniques and each being contained in a respective one of a plurality of sets of biological properties, the method including the steps of: converting the plurality of sets of biological properties into corresponding representations in a representation format which is invariant to the technologies used to derive the biological properties; determining, in said representation format, a representation from each of the converted sets of further biological properties which most closely matches the first representation of the first biological property; and re-converting the determined representations from the representation format back to the biological properties associated with the determined representations and thereby determining a correspondence between the first biological property and each of the further biological properties.

It will be appreciated that the first and second aspects share a number of common features and are primarily distinguished by the presence of at least three different sets of biological properties in the second aspect. The following optional and preferred features apply equally to both of the above first and second aspects.

As noted above, the present invention is particularly, but not exclusively, directed at determining correspondences between data derived from different single-cell analysis techniques (also referred to a single-cell 'omics techniques). Accordingly, at least one of the analysis techniques is preferably a single-cell analysis technique and more preferably all of the analysis techniques are single-cell analysis techniques.

An individual single-cell analysis technique is able to determine certain useful biological properties of the cells under analysis. However, due to the differing nature of the analysis techniques, the information that can be derived from each technique is different and thus each analysis technique can only provide a part of the complete biological picture of a particular cell or type of cell in a sample.

To obtain a clearer understanding of the biological properties of a particular cell or type of cell, it is advantageous to combine the results from different analysis techniques. However, the very nature of single-cell analysis means that it is only ever possible to perform one analysis technique on each individual cell. Therefore, if multiple analysis techniques are being performed on cells taken from a single sample, direct links between the respective biological properties are not available. With the aim of addressing this, the aspects of the present invention may allow correspondences to be identified between cells that have been analysed by one technique (and the biological properties thus determined) and cells that have been analysed by a different technique (and the biological properties thus determined). By pairing or matching cells analysed by one technique with a close (preferably the closest) "sibling" cell in the sample which has been analysed by another technique, the biological properties determined by both techniques can be linked and thus combined in further assessment or analysis.

Examples of single-cell analysis techniques are single-cell RNA sequencing ("scRNA-Seq"), Cytometry by Time-of-Flight (CyTOF) and Imaging Mass Cytometry ("IMC"), but the skilled person will appreciate that the methods of the above aspects are not limited to data derived using these tech1niques, and are equally applicable to other techniques (including techniques not currently know at the time of writing).

Similarly, whilst example biological properties may be a transcriptome and a proteome, the skilled person will appreciate that the methods of the above aspect are not limited to such properties and are equally applicable to other biological properties (including properties which are not derivable at the time of writing).

Preferably at least one of the biological properties is sequencing information determined by scRNA-Seq. At the time of writing, scRNA-Seq is the most detailed and information-rich single-cell analysis technique. However, each single-cell analysis technique only represents a partial observation of the properties of a cell. By combining information from more than one analysis technique using the correspondences identified by the methods of the above aspect, further information about the properties of a cell can be determined to build up a more complete picture of the cell.

The methods of the above aspects may proceed by assuming that cells share a common (low-dimensional) underlying structure and that the underlying distribution of cells having certain biological properties is approximately constant across the different analysis techniques. As a result a technology-invariant representation may be created, for example using an auto-encoder framework which may have an adversarial objective.

The methods may allow multi-modal datasets to be integrated by pairing cells across technologies using a matching scheme that operates on the low-dimensional representation.

The methods may use neural-networks and/or end-to-end training to produce the representation format, and/or a fast bipartite matching algorithm for the matching step. Either or both of these features can allow the methods to scale well in the number of cells in the input.

As samples are commonly used-up by individual analysis techniques (in particular single-cell analysis techniques), each sample which is subject to the analysis techniques in order to create the sets of biological properties for these methods may be divided into disjoint aliquots for analysis. Notwithstanding, given that the technology-specific datasets come from the same sample, i.e. cell mix, it can be expected that they exhibit the same underlying distribution. Therefore, the methods may assume a shared latent representation between technologies but, unlike other approaches, may not necessarily require one-to-one or overlapping correspondences between feature sets.

Further, as indicated in the second aspect above, the training scheme may allow for the addition of an arbitrary number of technologies, which can all be trained in parallel.

Preferably the representation format is used solely to match the cells. As a result the true observed marked abundances per cell pair, as measured with the different analysis techniques or technologies can be used in any downstream analysis.

The representation which is invariant to the technologies used to derive the biological properties may be a latent space.

In certain embodiments, the latent space may be constructed by creating neural networks for a) an encoder for each data set; b) a decoder for each data set; and c) a discriminator acting on the representations.

In certain embodiments a 2 layer architecture with 8 hidden units may be used as the encoder and/or decoder for one or more of the data sets. In certain embodiments a 2 layer architecture with 64 hidden units may be used as the encoder and/or decoder for one or more of the data sets.

A Gaussian activation may be used for one or more of the decoders.

The networks may be optimized using the ADAM optimizer.

The discriminator may be a binary classifier.

In certain embodiments the latent space may be constructed by minimizing the reconstruction error between the encoder and decoder for each data set while adversarially fooling the discriminator.

Whilst the representation format is preferably optimally integrated, the use of an optimal matching technique for determining the matching between the representations may compensate for even a sub-optimally integrated representation format. This may provide additional advantage over bare decoding.

The method may include the further step of determining a divergence score for the created representations in the representation format. The divergence score may be a Kullback-Leibler (KL) divergence or a variant thereof. In certain embodiments the divergence score may be calculated as

$$\widehat{D}(Z_s \parallel Z_t) = \frac{1}{2}\widehat{D}_{KL}(Z_s \parallel Z_t) + \frac{1}{2}\widehat{D}_{KL}(Z_t \parallel Z_s) \qquad (2)$$

where
$Z_s$ and $Z_t$ are the sets of codes and

$$\widehat{D}_{KL}(P \parallel Q) = \frac{d}{|P|}\sum_{p_i \in P} \log \frac{v_k(p_i)}{\rho_k(p_i)} + \log \frac{|P|}{|Q|-1}$$

where $v_k(p_i)$ and $\rho_k(p_i)$ are the distances from $p_i$ to the $k^{th}$ nearest neighbour in the sets $P$ and $Q$ respectively and all

$p_i \in \mathcal{R}^d$.

In certain embodiments, the method may include the further step of comparing the divergence score to a predetermined threshold. In certain embodiments if the divergence score exceeds the predetermined threshold, the step of converting the sets of biological properties into corresponding representations may be repeated until a divergence score is obtained which is equal to or less than the predetermined threshold.

Training of neural networks can be unstable and/or dependent on the initial choices. By checking the divergence of the representations in the representation format, it is possible to determine whether an acceptable representation format has been created.

Preferably, the step of determining uses a bipartite matching approach between each pair of the converted sets.

The step of determining preferably includes the sub-step of reducing the search space to reduce the number of possible correspondences, ideally prior to carrying out any matching. This can reduce the complexity of the matching step by reducing the number of potential matches for a particular representation. A reduced complexity can reduce the amount of computational power and/or time taken to complete the matching step. If the reduction is well chosen, then this can be achieved with only a very small, or preferably no appreciable, reduction in the accuracy of the matching.

The reduction in number of possible correspondences may be to a pre-determined maximum per matching, for example by using a k-Nearest Neighbour approach. The pre-determined maximum may vary depending on the size of the datasets, and will in each case be a trade-off between the accuracy of the matching and the reduction in the complexity.

However, it has been found that restricting the number of possible correspondences to no more than 50 can still result in high levels of accuracy in the matching. Greater numbers of possible correspondences, such as no more than 100, no more than 200, no more than 250 or no more than 500 provide further, but decreasing, increases in accuracy. Conversely restriction to no more than 2000, preferably no more than 1000, more preferably no more than 500 results in a significant reduction in the memory and CPU time required for the matching process. Accordingly, the pre-determined maximum is preferably between about 50 and about 2000 and more preferably between about 50 and about 500. Certain embodiments may restrict the number of matches to 50, 100, 200, 250 or 500.

In certain embodiments the bipartite matching approach uses the Euclidean distance in the representation format as the "cost", for example in generating a cost matrix, and seeks to find the combinations which result in the smallest overall cost.

In certain embodiments the bipartite matching uses the Jonker-Volgenant algorithm which has been shown to have good computational performance even in high-dimensional settings.

In alternative embodiments a minimum-cost maximum-flow algorithm may be used.

Many existing matching approaches (bipartite and otherwise) assume that there is a one-to-one correspondence between datasets. However, in the reality of cellular analysis and different analysis techniques this is unlikely to be the case. For example the cellular composition of the tissue samples examined by each analysis technique may differ. Alternatively or additionally the analysis techniques may be biased (for example, certain genes can be overrepresented in scRNA-Seq data whilst certain proteins could be overrepresented in CyTOF data). Alternatively or additionally the nature of the analysis techniques may mean that certain analysis techniques produce different numbers of data points from a given sample.

Therefore the matching processes preferably include further characteristics which adjust for and/or allow for this possibility.

In some arrangements, the step of determining allows for the possibility of a correspondence not being found by adding a null node to each converted set. The null node may be densely connected and preferably has a high capacity (e.g. the size of the (largest of the) opposing dataset). To bias the approach against selecting the null node unless no other realistic option exists, the null node is preferably given a high assignment cost. However, this null node will still capture correspondences with cells which are otherwise poorly-matched.

Alternatively or additionally, the step of determining may allow for many-to-one matches to representations in a converted set which has fewer elements. This may be done by increasing the capacity of edges in the nodes of the dataset with the smaller number of entries. Preferably the method also includes provision to penalise for any unmatched cells (whether to the null node or to a real node), for example by constraining the sum of the capacity of the smaller dataset(s) to the cardinality of the largest dataset.

These modifications to the matching process may be applied before or after the reduction in the number of possible correspondences.

The methods of the above aspects find particular application as part of an integrated platform providing clinical decision support, for example in the diagnosis of tumours in and the planning of treatments for patients with cancer. By allowing the integration of information obtained from a plurality of different analysis techniques, all of which have been performed on the same sample from the patient, an improved understanding of the tumour may be obtained and the appropriate clinical pathways indicated or chosen.

Further aspects of the present invention provide a computer program arranged, when run on a processor, to perform the method of the above first or second aspect, including some, all or none of the preferred or optional features of those aspects, and a computer program product having non-transitory memory storing such a computer program.

Clinical decision support systems are used at the point-of-care, in particular by clinicians, to analyse and visualize information acquired by different (diagnostic) analysis techniques and to facilitate combination of the acquired data with the clinician's knowledge and expertise. More and more such clinical decision support systems are developed which seek to leverage data from high-throughput next-generation analysis techniques such as whole genome sequencing (WGS) and total RNA sequencing (RNA-Seq).

State of the art single cell analysis techniques, e.g. scRNA-Seq and CyTOF, retain a correlation between individually measured data points and the cell from which the analysis material derived throughout the analysis. For example, this may be achieved by barcoding the molecules deriving from one cell in a heterogeneous sample and reading the barcode information together with the respective data point of the biological property e.g. a (barcoded) mRNA molecule.

However, if multiple analysis technologies are used to analyse the same patient sample (e.g. tumour sample) no correspondence between individual cells is retained between the different techniques. The present invention provides a method of determining such correspondences between different biological properties (e.g., transcriptome and proteome) on a single-cell level.

Methods of the present invention can, for example, be used to aggregate data from different single-cell analysis techniques in a clinical decision support system. The clinical decision support system may present a visualization of the aggregated data to support the clinician's decision how to treat a patient based on the molecular mark-up of the patient's tumour sample.

Alternatively or additionally, the clinical decision support system may propose treatment options to the clinician based on the aggregated data alone or in combination with clinical and medical history data.

A further aspect of the present invention provides a clinical decision support computer system having a processor which is arranged to perform the method of the above first or second aspect, including some, all or none of the preferred or optional features of those aspects. The computer system may have a display to allow the results of the determined correspondence(s) to be displayed to a user.

Further aspects of the present invention provide a clinical decision support computer program arranged, when run on a processor, to perform the method of the above first or second aspect, including some, all or none of the preferred or optional features of those aspects, and a clinical decision support computer program product having non-transitory memory storing such a computer program.

Embodiments of the present invention will now be described by way of non-limitative examples with reference to the accompanying drawings, in which:

Fig. 1 shows, schematically, a method according to an embodiment of the present invention which performs a pairwise matching of cell across multiple single cell analysis technologies;

Fig. 2 shows a schematic matching graph structure forming part of a method according to an embodiment of the present invention;

Fig. 3 shows the tree used to simulate a PROSSTT dataset used to test a method according to an embodiment of the present invention;

Fig. 4 shows the tree used to simulate a further PROSSTT dataset used to test a method according to an embodiment of the present invention and a density plot of the pseudotime label between a pair of cells matched between source and target technologies;

Fig. 5 is a tSNE (perplexity=32) plot of the integrated latent space for the simulated data derived from the tree structure shown in Fig. 3;

Fig. 6 is a density plot of the pseudotime label in the simulated data between a pair of cells matched between source and target technologies;

Fig. 7 is tSNE (perplexity=32) plot of the integrated latent space for the simulated data derived from the tree structure shown in Fig. 4;

Fig. 8 is a density plot of the pseudotime label between a pair of cells matched between source and target technologies using latent codes obtained from MATCHER, a prior art system;

Fig. 9 is a tSNE embedding (perplexity=128) of the integrated latent space created by the application of a method according to an embodiment of the present invention to datasets derived from human tumour cells, with cell matches indicated by the grey lines;

Fig. 10 is a series of density plots of HLA-DRA marker abundance measured with scRNA-Seq (gene, x-axis) and CyTOF (protein, y-axis);

Fig. 11 shows a comparison of matching accuracy, with respect to cell-type label, between sparse and dense connections when a method according to an embodiment of the present invention is applied to datasets derived from human tumour cells;

Fig. 12 is a tSNE embedding similar to that of Fig. 6 but with cell matches obtained via matching on the data space indicated by the grey lines; and

Fig. 13 is a series of charts showing the training progress of a method according to an embodiment of the present invention on the datasets derived from human tumour cells.

An embodiment of the present invention which provides a method of matching cells from a source technology to cells in a target technology will be described below. The method has been tested on both simulated and real data networks as explained further below.

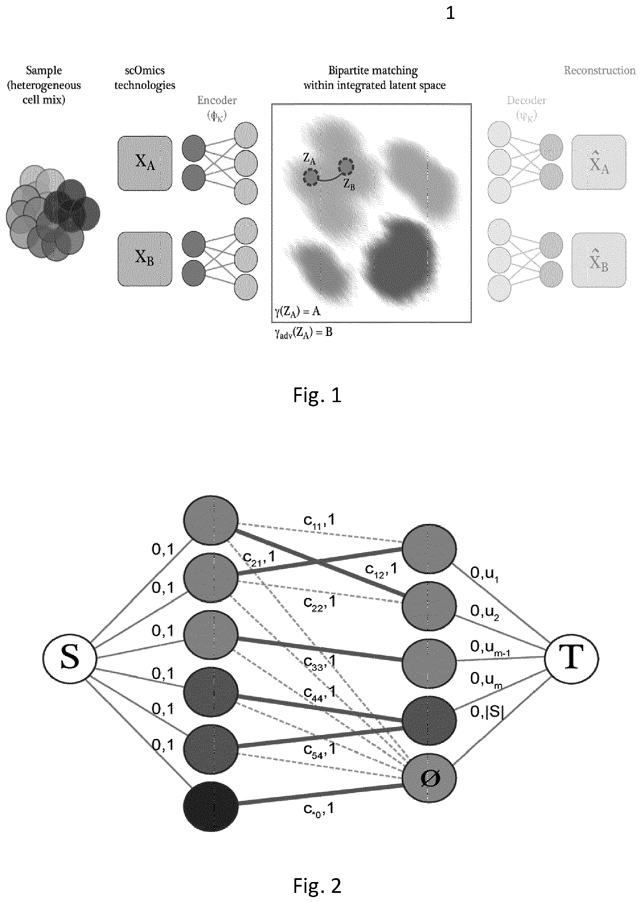

Figure 1 illustrates the formation and operation of the method of this embodiment. The method assumes that the input of each technology comes from a similar distribution of cells, for example from a common sample, processed in parallel. The method proceeds by mapping cells into a technology-invariant latent space using an auto-encoder framework as described further below and an adversarial objective, such that the original cell signal can be faithfully reconstructed from the latent space, while the technology of each latent representation is indistinguishable. The obtained shared latent codes are then used to find the best cell analogues across technologies by using a fast bipartite matching framework.

*Constructing a Technology-Invariant Latent Space & Model Architecture*

An integrated latent space has ideally two properties. It must be possible to decode the latent representations into faithful reconstructions of their inputs, while the source technology of each latent representation should be indistinguishable. To this end, the method of the present embodiment has three types of networks: a pair of encoder ($\phi_k$) and decoder ($\psi_k$) networks for each technology k, which map into and reconstruct out of the latent space, and a single discriminator network ($\gamma$) acting on the latent representations.

The discriminator is a binary classifier trained to identify the latent representations of a source technology from latent representations of all other technologies. The discriminator uses binary cross entropy.

The method of the present embodiment creates an integrated latent space by minimizing the reconstruction error while adversarially fooling the discriminator. Given the measurements of a batch of cells from the target technology,

$x_t$ and the (fixed) latent representations of a batch of cells from the source technology, $z_s = \psi_s(x_s)$ the method minimizes the following objective function:

$$\mathcal{L}(x_t, z_s; \phi_t, \psi_t) = \mathcal{L}_{nll}(x_t, \hat{x}_t; \phi_t, \psi_t) + \beta\mathcal{L}_{adv}(z_s, z_t; \phi_t) \tag{1}$$

where $\hat{x}_t = \phi_t(z_t)$ is the reconstruction of $x_t$ and $\mathcal{L}_{nll}(x_t, \hat{x}_t)$ is the negative log-likelihood of the inputs under the reconstruction. For a Gaussian likelihood the latter has the form $\mathcal{L}_{nll}(x_t, \hat{x}_t) = \|x_t - \hat{x}_t\|_2^2$. $\mathcal{L}_{adv}$ is the discriminator's classification error when trying to classify $z_s/z_t$ as the target/source technology. $\beta$ is a hyperparameter weighing the influence of the adversarial loss.

All networks use the ReLU activation. The latent dimension of all models is set to 8, and discriminator networks with 2 layers and 8 hidden units each are used. The SNGAN (Miyato et al., 2018) framework is used to train the discriminator.

For the simulated data networks discussed further below, a 2 layer architecture with 64 hidden units for was used. For the real data networks, a 2 layer architecture with 8 hidden units was used for the CyTOF networks, and a 2 layer architecture with 64 hidden units used for the scRNA-Seq networks. A Gaussian activation was used for all decoders. Architecture search was done on VAE codes and reflects a trade-off between number of features and number of parameters.

Optimization was carried out by iteratively fixing one technology as the source and one technology as the target. In the case of more than two technologies, the technology corresponding to the discriminator's positive class must either be the source or target technology. The codes of the source technology are fixed and equation (1) is minimized with gradient updates to the encoder and decoder, $\phi_t$ and $\psi_t$, of the target technology using gradients computed on the batch $x_t$. After each update, the discriminator is trained on $z_s$ and $z_t$. The method is initialized by first training a VAE (Kingma and Welling, 2013) on a single technology, and using the latent representations as the first set of $z_s$. All networks are optimized using the ADAM (Kingma and Ba, 2014) optimizer.

Due to the min-max nature of adversarial training, model comparison is challenging since it is not possible to directly compare the minimized objective functions of converged models (Lucic et al., 2017). The computer vision community has introduced a number of metrics specific to the image domain to help compare models (Heusel et al., 2017; Salimans et al., 2016), however, and these can be used to validate the quality of a set of lower dimensional latent representations. As was done in Wang et al. (2019), a k-Nearest Neighbor (kNN)-based divergence estimator (Wang et al., 2009) is used to quantitatively evaluate the quality of the integrated latent space. The divergence score between two sets of codes $Z_s$ and $Z_t$ is calculated as:

$$\widehat{D}(Z_s \parallel Z_t) = \frac{1}{2}\widehat{D}_{KL}(Z_s \parallel Z_t) + \frac{1}{2}\widehat{D}_{KL}(Z_t \parallel Z_s) \tag{2}$$

where

$$\widehat{D}_{KL}(P \parallel Q) = \frac{d}{|P|}\sum_{p_i \in P} \log\frac{v_k(p_i)}{\rho_k(p_i)} + \log\frac{|P|}{|Q| - 1}$$

where $v_k(p_i)$ and $\rho_k(p_i)$ are the distances from $p_i$ to the $k^{th}$ nearest neighbour in the sets P and Q respectively and all $p_i \in \mathcal{R}^d$.

This estimator approximates a symmetric variant of a Kullback-Leibler (KL) divergence, a measure of how much two distributions differ, using only empirical data. The divergence estimate is computed between the latent representations of the source technology and the target technology.

*Matching*

The obtained shared latent representation can then be used for finding analogous cells across technologies, i.e. those cells in the samples analysed by each technology which are most closely related to each other. Each cell is characterized in the latent space by a low-dimensional vector of latent codes, which are in one-to-one correspondence across technologies. To find the optimal pairwise matching in a computationally efficient way, the task can be viewed as a combinatorial bipartite matching problem.

However, given the high-dimensional nature of single-cell data, as a first step it is helpful to reduce the search space to the most-likely potential matches. In order to achieve this, a k-Nearest Neighbours (kNN) graph is built using source data and then queried by the target technology cells. The nodes correspond to cells and edge weights correspond to the distance between the cells. Since the latent codes are dense and directly comparable between the technologies, the Euclidean distance is used to measure the dissimilarity between each pair of cells. The sparsity of connections, regulated by the choice of hyperparameter k, corresponds to the trade-off between the computational performance (memory usage, run time) and the matching accuracy.

Given a cost matrix (e.g., Euclidean distance in latent or data space) with distances between all pairs of cells across technologies, the aim is to find row-cell pairs that result in the smallest overall cost. In the present embodiment, Euclidean distance in the latent space is used to generate the cost matrix. The aim then corresponds to solving a Linear Assignment Problem (LAP), i.e. bipartite matching, and can be formulated as:

$$\min_{i,j} \sum_i \sum_j C_{ij} X_{ij} \tag{3}$$

with cost matrix C, Boolean assignment matrix $X$ and cell indices $i \in \{1, \ldots, n\}$, $j \in \{1, \ldots, m\}$, where n, m denote the number of cells in source and target dataset, respectively. In the classical bipartite matching n=m and when formulated as a graph with cellular nodes, the capacity of each edge is exactly one, as bijective matching is enforced. To efficiently solve a LAP with sparse connections we use the Jonker-Volgenant Algorithm (Jonker and Volgenant, 1987), which has been shown to have good computational performance even in high-dimensional settings (Dell'Amico and Toth, 2000), despite the worst-case complexity of $O(n^3)$.

To relax the restriction on only one match, the generalized framework of Minimum-Cost Maximum-Flow Problems (Ahuja et al., 1993; Klein, 1967) can be applied.

Given a directed graph $G = (V, E)$, with $V$ denoting vertices and $E$ edges, the Minimum-Cost Maximum-Flow of $G$ would be the maximum flow that can be pushed from source to sink at a minimum cost. If $u$ denotes the non-negative edge capacities and $c$ the corresponding costs, then the flow of $f(v, w)$ units on edge $(v, w)$ would contribute $c(v, w).f(v, w)$ to the objective

$$\min_{(v,w)} \sum_{(v,w)} c(v,w) f(v,w) \tag{4}$$

subject to

$$0 \leq f(v,w) \leq u(v,w) \qquad \forall_{(v,w) \in E} \qquad (5)$$

$$\sum_{w \in V} f(v,w) - \sum_{w \in V} f(w,v) = b(v) \qquad \forall_{v \in V} \qquad (6)$$

Finding a minimum cost of the flow through the network corresponds to finding the shortest path. Many algorithms have been designed to efficiently solve the Minimum-Cost Maximum-Flow Problem in polynomial time (see, for example, Ahuja et al., 1993; Kovács, 2015).

The approach described so far makes a strong assumption of observing the same cellular composition across datasets, i.e., each cell has a direct corresponding sibling in the other technology. In order to allow for mismatches due to expected variation in cellular composition, the kNN graph is expanded with sparse connections by adding a densely-connected null node with high capacity and high assignment cost, capturing the otherwise poorly matched cells.

This graph structure is depicted in Figure 2 in which the latent space is depicted as a set of nodes corresponding to cells in the source (S) and target (T) datasets and edges to the sparse connections between them, resulting from a kNN search. Edge labels (a, b) indicate matching cost and edge capacity, respectively. The null node, colored in grey, bottom-right, captures matches with source cells that lack a close enough analogue in the target technology. Its capacity equals the cardinality of the source data and the cost $c_{*0}$ is relatively high. The thicker lines linking the nodes represent the actual matches selected by the algorithm.

Furthermore, to account for differences in the number of cells between modalities, many-to-one matches are allowed by increasing the capacity of the outgoing source edges, assuming the source corresponds to the smaller dataset. In order to penalize for leaving cells unmatched, the sum of outgoing source capacities, excluding the null node, is constrained to equal the cardinality of the target set

$$\sum u_i = |T| \qquad i \in \{1, \dots, m\} \quad (7)$$

The quality of matching is evaluated on different levels. First, the accuracy corresponding to the fraction of true positives w.r.t. cell-type label is reported. Cell-types can be determined in a technology-specific manner. If more fine-grained cellular information is available, such as pseudotime, a direct comparison of this quantity is carried out.

*Simulated Dataset*

The method of the present embodiment was first evaluated using two synthetic datasets generated by PROSSTT (Papadopoulos et al., 2019). PROSSTT simulates a temporal branching process that parameterizes a negative binomial model.

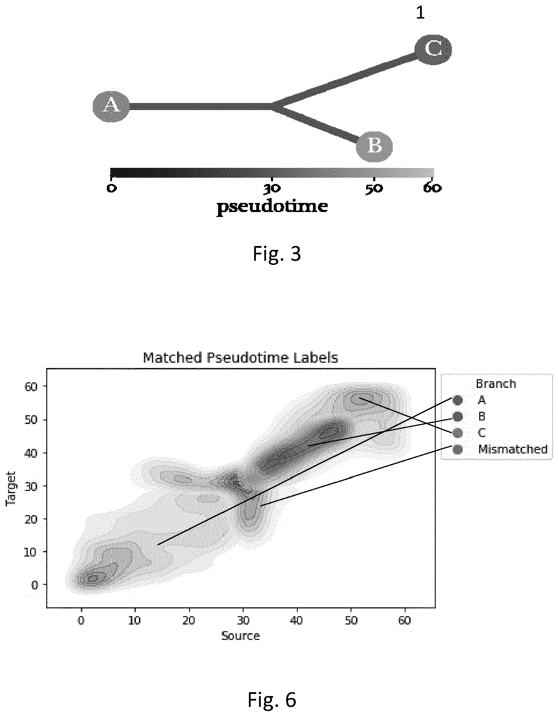

Two single cell analysis technologies were simulated by running PROSSTT under different seeds and different numbers of genes but retaining the underlying branching structure (see Table 1). Thus these two datasets have a common latent structure yet their features do not have any correspondences. A three branch tree as illustrated in Figure 3 was used with different branch lengths. The first branch A runs from pseudotime 0 to 30, branch B from 30 to 50 and branch C from 30 to 60. The PROSSTT simulated data allows an underlying latent structure to be defined.

Table 1. Characteristics of PROSSTT simulated dataset using different seeds but the same underlying latent structure.

| Dataset | # Markers | # Cells |
|---------|-----------|---------|
| Source | 256 | 32,000 |
| Target | 128 | 32,000 |

The method of the present embodiment was run using the larger dataset (i.e. the one with more markers) as the source technology and the smaller dataset as the target technology. The latent space was initialized by training a VAE on the source technology for 250 epochs. These latent representations are fixed, and then the target technology was trained for 250 epochs. To help orient the latent space, branch labels were given to the discriminator (Makhzani et al., 2015). For each target cell a set of its most similar cells in the source dataset was identified by k-Nearest Neighbors algorithm with k=500, using the Euclidean distance on the latent codes. The optimal matching was then found by solving the Minimum-Cost Maximum-Flow problem, allowing for many-to-one matches.

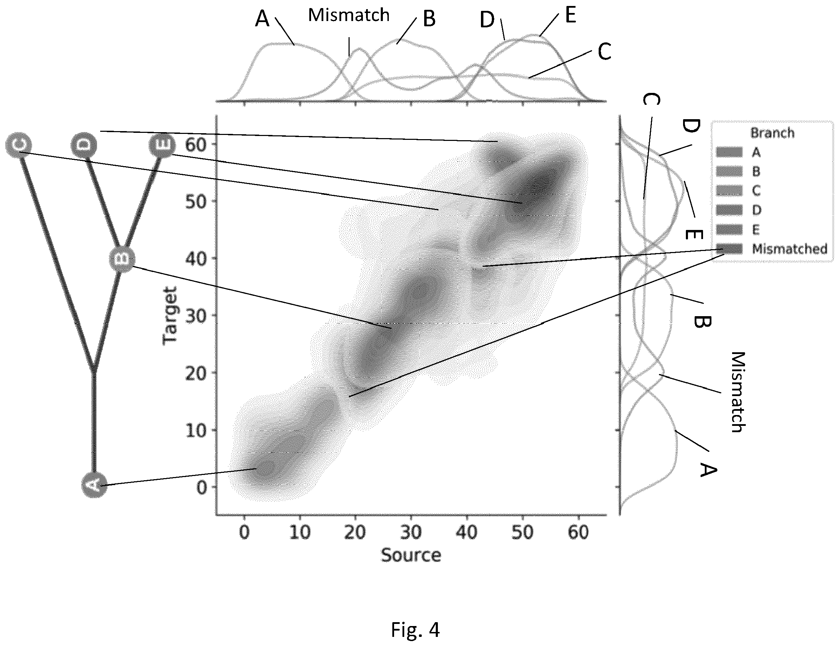

In a second approach, three single cell analysis technologies were simulated by running PROSSTT under different seeds but retaining the underlying branching structure. Thus these three datasets also have a common latent structure yet their features do not have any correspondences. A five branch tree, shown on the left hand side of Figure 4, was used with different branch lengths. Each dataset contained 64,000 cells with 256 markers.

The method of the present embodiment was run on the three simulated datasets. The latent space was initialized by training a VAE on the source technology for 256 epochs. These latent representations were fixed, and then the two target technologies are trained for 256 epochs. For each target cell a set of its most similar cells in the source dataset were identified by k-Nearest Neighbors algorithm with k=500, using the Euclidean distance on the latent codes. The optimal matching was then found by solving the bipartite matching problem.

Results

The method of the present embodiment was evaluated on the above pair of datasets generated by PROSSTT, such that there are no feature correspondences between the two datasets, while the shared underlying characteristics are preserved. Branches in PROSSTT define over-arching structure that mimics cell-types, while the temporal component, i.e., pseudotime, provides a continuous interpolation from one branch to another, as defined by the tree.

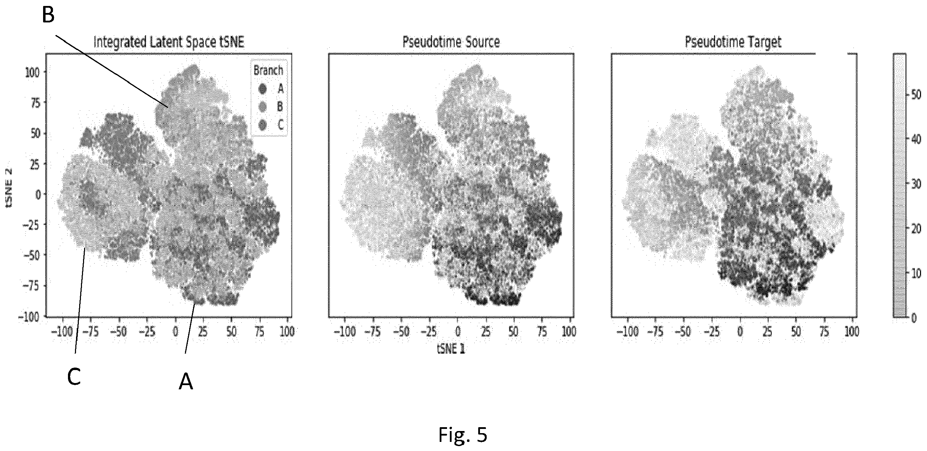

In latent space, the branch structure within the data produces large clusters, while the pseudotime structure provides orientation within each cluster as well as global smoothing of the manifold. For the three branch tree, the method of the present embodiment was able to correctly capture this structure as well as orient it across datasets, as shown in tSNE embeddings computed on the combined latent representations from both sources as shown in Figure 5.

Each point in Figure 5 corresponds to a cell's latent representation. In the first axis, points are coloured by the branch label and shaded by the source technology. Figure 5 shows that the tree's branching structure is represented in the embedding and that the technology codes are grouped globally by branch label. In the second and third axes the source/technology codes are coloured by pseudotime label. As illustrated, the method was able to correctly capture the intra-cluster orientation.

The optimal matching algorithm was applied to a sparse kNN graph with k=1500. Figure 6 provides a quantitative evaluation of the matches with respect to pseudotime. Coloured contours correspond to cells paired within the same branch. The grey contour corresponds to cells paired to a different branch. It can be seen that this mismatching mostly happens around the branching point, where cells from different branches are most similar.

The highest density of the cells is observed within a close proximity of the diagonal, thus showing that the derived matches do not only follow the overall branches but also reflect subtle changes. The mismatched cells (grey colour), w.r.t. branch label, constitute 15% of the total cell population.

Table 2 below is the corresponding confusion table.

Table 2. Confusion table showing branch labels of the optimal matches of cells in the simulated PROSSTT data. Entries on the diagonal correspond to correct matches whereas off-diagonal elements are mismatches. The overall accuracy, with respect to branch label, is 85%.

| Target Source | A | B | C |
|---|---|---|---|
| A | 10007 | 1290 | 612 |
| B | 1419 | 6277 | 332 |
| C | 858 | 156 | 11049 |

The majority of the mismatches is located around the branching point, where the differences between cells become indistinguishable. As expected, the overall accuracy of the matches increases if the area of the branching point (pseudotime $\in [30-E, 30+E]$) is not taken into account (accuracy: 98%, Table 3 below). The Spearman's and Pearson's correlation coefficients between all the matches amount 0.89 and 0.87, respectively. These results demonstrate that the method of the present embodiment is capable of accurately identifying the best matching cells across technologies, based on the shared latent representations, even in the absence of paired features.

Table 3. Confusion table showing branch labels of the optimal matches of cells in the simulated PROSSTT data, but excluding the branching point (pseudotime=30) +/- epsilon of 10% of total pseudotime ($E = 0.1 * 60 = 6$). Entries on the diagonal correspond to correct matches whereas off-diagonal elements correspond to mismatches. The overall accuracy, with respect to branch label and with branching points excluded, is 98%.

| Target Source | A | B | C |
|---|---|---|---|
| A | 7345 | 112 | 1 |
| B | 212 | 4301 | 6 |
| C | 120 | 1 | 8410 |

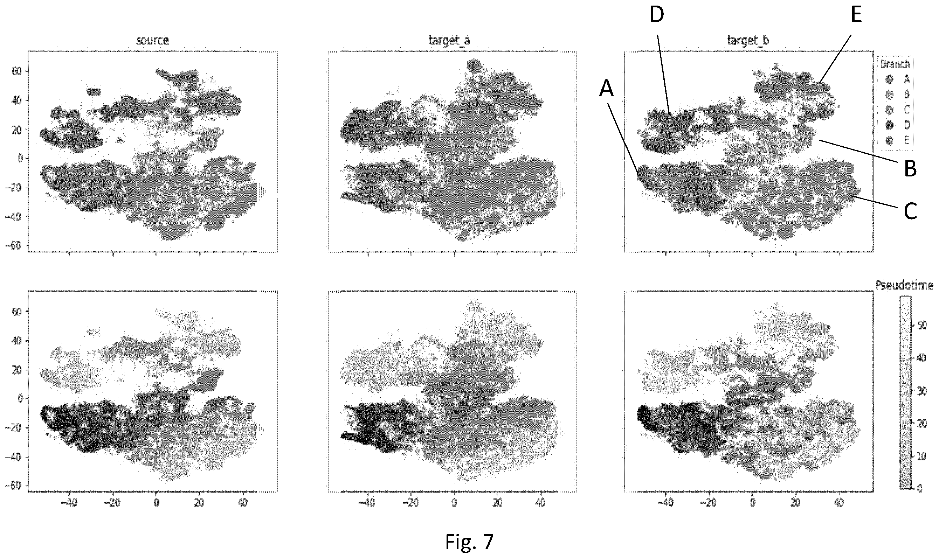

For the five branch dataset, the method of the present embodiment was also able to correct capture the underlying data structure as well as orient it across the datasets as shown in tSNE embeddings computed on the combined latent representations from all datasets as shown in Figure 7.

The optimal matching Minimum-Cost Maximum-Flow algorithm described above was applied to a sparse kNN graph with k=50. The plot on the right hand side of Figure 4 provides a quantitative evaluation of the matches with respect to pseudotime.

The highest density of the cells is observed within a close proximity of the diagonal, thus showing that the derived matches do not only follow the overall branches but also reflect subtle changes. The mismatched cells (grey color), with respect to branch label, constitute 15% of the total cell population. The corresponding confusion table is shown as Table 4 below.

The majority of the mismatches are located around the branching points, where the differences between cells become indistinguishable. The Spearman's and Pearson's correlation coefficients between all the matches amount 0.83 and 0.86, respectively. These results demonstrate that the method of the present embodiment is capable of accurately identifying the best matching cells across technologies, based on the shared latent representations, even in the absence of paired features.

Table 4. Confusion table showing branch labels of the optimal matches of cells in the simulated PROSSTT data (5 branches). Entries on the diagonal correspond to correct matches whereas off-diagonal elements correspond to mismatches. The overall accuracy, with respect to branch label, is 86%.

| Target Source | A | B | C | D | E |
|---|---|---|---|---|---|
| A | 8528 | 583 | 679 | 99 | 23 |
| B | 724 | 6285 | 758 | 466 | 928 |
| C | 770 | 777 | 18917 | 48 | 99 |
| D | 42 | 371 | 22 | 7804 | 266 |
| E | 28 | 1086 | 41 | 305 | 7363 |

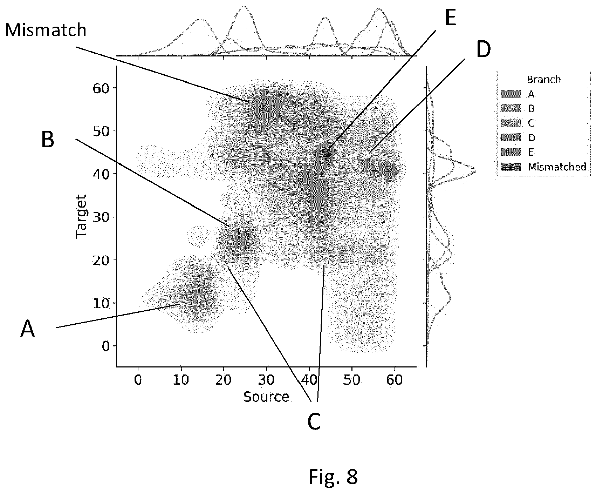

The method of the present embodiment was also compared to the MATCHER approach disclosed in Welch et al., 2017. MATCHER assumes a one-dimensional latent structure, which is violated by the branching structure in the simulated data. Additionally, MATCHER is a probabilistic model based on Gaussian processes and is thus limited in terms of scalability. To this end a budget of 48 hours compute time with maximum of 40 Gb memory was set. After this, the matching step of the present embodiment was applied to find matches using a kNN graph with k = 50 and null node cost of 95. Figure 8 shows a density plot of the pseudotime lable between a pair of cells matched between source and target technologies using latent codes obtained from MATCHER. Colored contours correspond to cells paired within the same branch, while the grey contour corresponds to cells paired to a different branch. The accuracy with respect to the branch label was 4%, while 40% of the cells were matched to the null node, since a better match could not be identified.

*Single-cell profiles of a melanoma patient*

The real dataset used to further exemplify the operation of the method of this embodiment is generated by the Tumor Profiler (TuPro) Consortium (Irmisch et al., 2020) as part of a multi-center, multi-cancer study comprising metastatic tumors from a cohort of deeply phenotyped individuals. Each patient's data is analyzed with multiple technologies, including scRNA-sequencing (Tang et al., 2009), Cytometry by Time Of Flight (Bandura et al., 2009, CyTOF) and Imaging Mass Cytometry (Giesen et al., 2014, IMC). All of these are capable of dissecting the tumor microenvironment and providing single-cell level, complementary information about the sample of interest. Although cell identity is lost throughout the process, the cells investigated by both technologies stem from the same population (i.e., were obtained from an aliquot of a common cell suspension).

For the CyTOF dataset, the patient's sample was profiled with CyTOF using a 40-markers panel designed for an in-depth characterization of the immune compartment of a sample. Data pre-processing was performed following the workflow described in Chevrier et al., 2017, 2018. Cell-type assignment was performed using a Random Forest classifier trained on multiple manually-gated samples. To investigate the utility of the method of the present embodiment, a subset of B Cells and T Cells only was considered, comprising m=135,334 cells. This dataset is referred to below as the target dataset.

A second aliquot of the same patient sample was analyzed by droplet-based scRNA-sequencing using the 10x Genomics platform. Standard QC-measures and pre-processing steps, such as removal of low quality cells, as well as filtering out mitochondrial, ribosomal and non-coding genes, were applied. Expression data was library-size normalized and corrected for the cell-cycle effect. Cell-type identification was performed using a set of cell-type-specific marker genes (Tirosh et al., 2016). Genes were then filtered to a set of 256, retaining those that could code for proteins measured in CyTOF channels, the top 32 T Cell /B Cell marker genes, and the remaining most variable genes. The total number of B Cells and T Cells in this dataset amounts to n=4,683. The scRNA-Seq dataset is used as the source dataset in the examples below.

Table 5. Characteristics of TuPro patient datasets.

| Dataset | # Markers | # Cells | T Cells | B Cells |
|---|---|---|---|---|
| scRNA-Seq | 1,024 | 135,334 | 70% | 30% |
| CyTOF | 41 | 4,683 | 73% | 27% |

A 2-layer encoder and decoder was used for the scRNA-Seq / CyTOF technology, with 64/8 units in each hidden layer respectively. The discriminator was a 2 layer network with 8 units and the latent dimension was set to 8. All networks used the ReLU activation. The discriminator was conditioned with the cell type label. These were trained for 256 epochs. Identification of cell analogues between scRNA-Seq and CyTOF data, downsampled to the size of scRNA-Seq data (m=n=4,683 cells) is based on a sparse kNN graph, with k=500, computed using Euclidean distance.

Results

The method of the present embodiment was applied to integrate scRNA-Seq and CyTOF datasets generated from a single sample by the TuPro project as discussed above. Integrating these technologies (and other single-cell analysis techniques) can allow for a multi-view perspective on cell dynamics and thus can lead to a more thorough understanding of the undergoing biological processes.

The optimal matching on the latent codes has a 97% accuracy to recover the cell-type label. In comparison, matching on the data space for the same cells would result in only 72% accuracy with respect to the cell-type label.

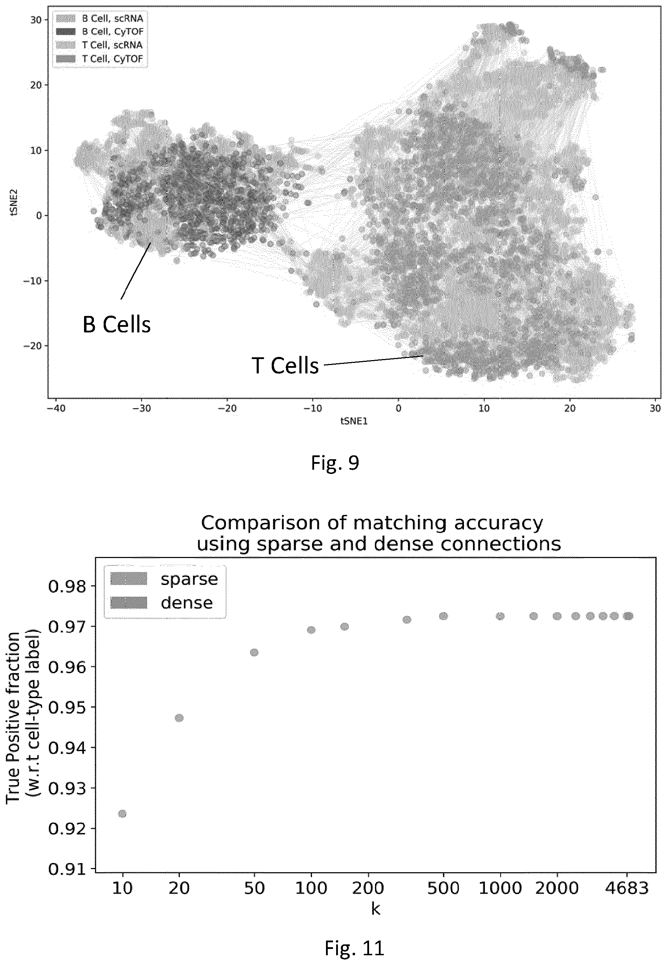

A more fine-grained visual evaluation is performed by inspecting the matches on a tSNE embedding of the integrated latent space shown in Figure 9, marked by grey lines. The colours (blue, orange) represent cell-types (B Cell, T Cell) and colour shades correspond to the profiling technology (light: scRNA-Seq, dark: CyTOF). The data has been downsampled at random to the size of the scRNA-Seq data (n = 4.683 cells).

Given different cell-type proportions in the data, a certain percentage of mismatches, in this case 3%, is expected, which corresponds to the lines joining points across the two cell types. Note that, for this visualization and to investigate and demonstrate the accuracy of the method, no null node was included in the graph, which would have captured most of the mismatches.

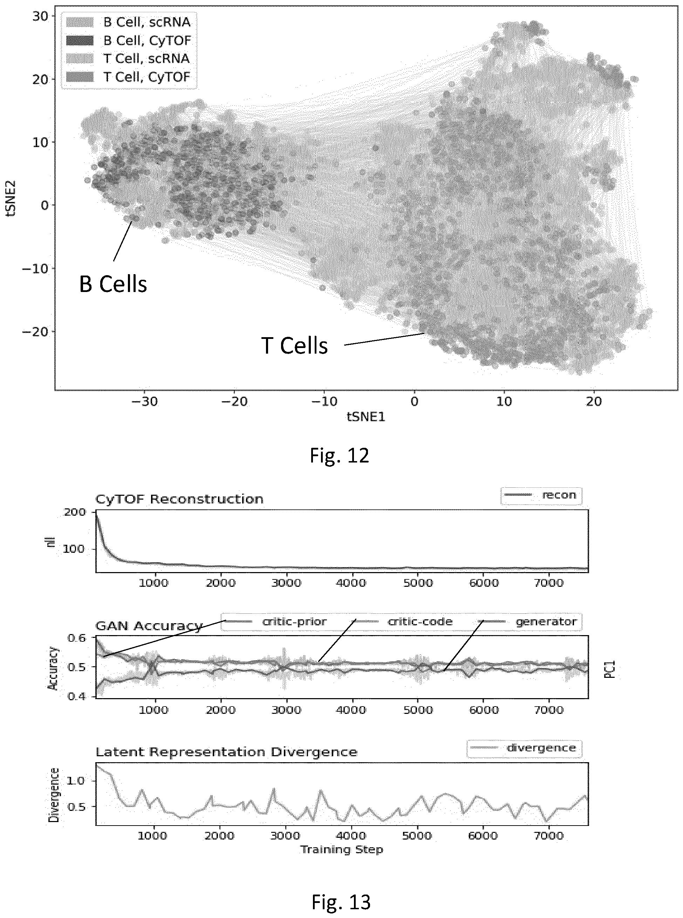

The equivalent plot using matching directly on the data space is shown in Figure 12. With cell matches obtained via matching on the data space indicated by the grey lines. Colours (blue, orange) represent cell types (B Cell, T Cell) and colour shades correspond to the profiling technology (light: scRNA-Seq, dark: CyTOF). The data has been downsampled at random to the size of scRNA-Seq data (n=4.683 cells). The accuracy of such matching is inferior to that using the latent representations and thus, many more links across the cell types can be observed.

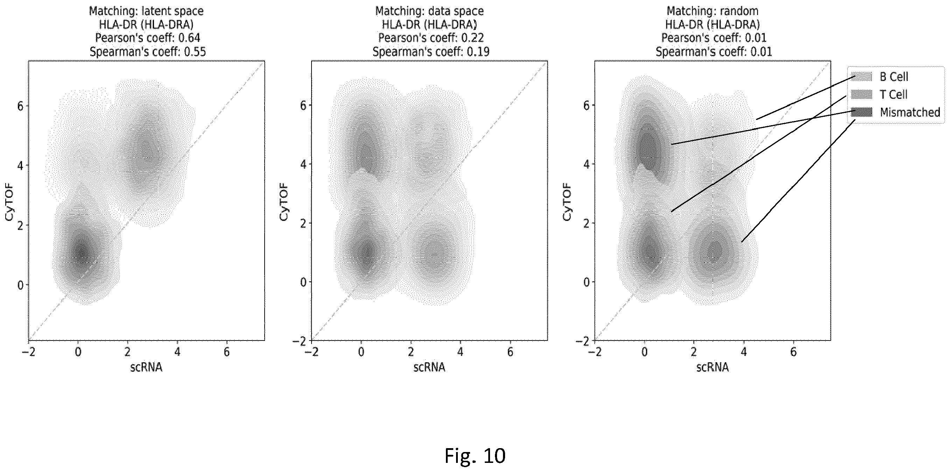

Further, a more fine-grained information of marker expression correlation was used to quantitatively assess the matching quality in both cases. This used the correlation coefficients between the expression of a cancer-relevant HLA-DRA gene and the corresponding HLA-DR protein abundance, with the bivariate density plots shown in Figure 10 with the HLA-DRA marker abundance measured with scRNA-Seq (gene, x-axis) and CyTOF (protein, y-axis). Coloured contours (bottom left, top right) correspond to cells paired within the same cell-type. The grey contour (top left, bottom right) corresponds to cells paired to a different cell-type. The colour intensity is weighted by the cell-type proportions. The plots correspond to, from left to right, matching in latent space, matching in data space and matching at random. Spearman's and Pearson's correlation coefficients are indicated for each plot and clearly show the benefits of performing optimal matching (matching at random has the lowest correlation coefficients) and at using shared latent representations for this task (highest correlation coefficients).

These show that matching using shared latent representations is significantly superior to using common features in data space (Pearson's coefficients: 0.64 and 0.22, respectively). Moreover, in both cases the optimal matching led to an improved expression correlation in comparison to matching the cells between the two technologies at random (Pearson's coefficient of 0.01). Thus, even in the presence of a subset of paired features across the technologies, using the shared latent representations proves beneficial for finding cell analogues.

A universal divergence estimator (Wang et al., 2009) was used, as described above, to evaluate the quality of the integrated latent space. Figure 11 shows the training progress of a method according to the embodiment of the present invention on the cancer patient sample. A VAE was trained on scRNA-Seq data and a method according to the embodiment described above used to integrate CyTOF representations into the latent space defined by the scRNA-Seq codes.

Figure 13 shows several metrics as the model trains. The top panel shows the negative log-likelihood of the CyTOF reconstruction. The middle panel shows the performance of the discriminator to correctly classify scRNA-Seq codes (critic-prior), CyTOF codes (critic-code) and the ability of the encoder to fool the discriminator, i.e. the misclassification accuracy (generator). The bottom left panel shows the divergence of the latent representations. To measure the degree of integration of the latent space, the estimated divergence is computed on the latent representations of the two technologies, and the optimization is deemed to be a success if the divergence and reconstruction error are below empirically-set thresholds (divergence < 0.3, $\mathcal{L}_{nll} < 47$). It was found that performance depended on tuning $\beta$ and the learning rates of the encoder and critic networks as shown in Table 6 below. The most stable hyperparameter setting employs a large weight on the adversarial gradients, $\beta = 512$, with a critic learning rate of $1 \times 10^{-3}$, and an encoder/decoder learning rate of $5 \times 10^{-4}$.

Table 6. Ablation study for training a method of the present embodiment on a real tumor sample. $\beta$ is the regularization strength of the adversarial loss. Learning rates are the initial settings of the ADAM optimizer. If the latent space divergence (Wang et al., 2009) is below 0.3 and the negative log likelihood of the input under the reconstruction is below 47, the training is deemed a success. These values were chosen empirically. $\beta$ and optimizer learning rates were heavily influential on model success.

| | | Learning Rate | | | |
| $\beta$ | Discriminator | Encoder/Decoder | Success | Replicates |
| --- | --- | --- | --- | --- |
| 512 | 0.0010 | 0.0005 | 30% | 10 |
| 128 | 0.0005 | 0.0005 | 12% | 8 |
| 64 | 0.0010 | 0.0010 | 12% | 8 |
| 64 | 0.0005 | 0.0010 | 11% | 9 |
| 256 | 0.0010 | 0.0005 | 10% | 10 |
| 256 | 0.0005 | 0.0005 | 7% | 14 |
| 512 | 0.0005 | 0.0010 | 0% | 9 |
| 512 | 0.0005 | 0.0005 | 0% | 4 |
| 512 | 0.0010 | 0.0010 | 0% | 3 |
| 256 | 0.0010 | 0.0010 | 0% | 8 |
| 256 | 0.0005 | 0.0010 | 0% | 6 |
| 128 | 0.0010 | 0.0005 | 0% | 5 |
| 128 | 0.0010 | 0.0010 | 0% | 6 |
| 128 | 0.0005 | 0.0010 | 0% | 1 |
| 64 | 0.0010 | 0.0005 | 0% | 6 |
| 64 | 0.0005 | 0.0005 | 0% | 3 |
| 32 | 0.0005 | 0.0005 | 0% | 5 |
| 32 | 0.0005 | 0.0010 | 0% | 2 |
| 32 | 0.0010 | 0.0010 | 0% | 4 |
| 32 | 0.0010 | 0.0005 | 0% | 7 |

Bipartite matching was chosen since it ensures a global optimal matching between two technologies across all cells. Due to the large number of cells profiled with each individual technology per sample as well as the nature of the cell matching problem, as described above, a kNN search was combined with a bipartite matching. A comparison of the accuracy is shown in Figure 11 in which the number of considered Nearest Neighbors k is indicated on the x-axis, which is displayed on a log10-scale. The fraction of True Positive matches, with respect to cell-type label, is depicted on the y-axis. The accuracy level of dense connection is achieved already for $k = 500$ and is within 1% absolute difference for $k = 100$.

Thus, comparing the bipartite matching alone to the combined approach shows that equivalent accuracies can be achieved using a neighbourhood of only k=500 for the total of 4,683 cells. This is in line with expectations, since a match to an extremely distant neighbor could only be justified in case of many multiple ties, i.e., a set of indistinguishable cells. Thus, matching in the search space reduced to the closest neighborhood is correspondent to finding cell analogues based on the dense distance matrix.

Further, solving the task using the combined approach is computationally efficient, as with 0.5Gb of memory usage and less than 1min of compute the pairwise matches for all the 4,683 cells were found, using k=500. As expected, with increasing k more computational resources are required to solve the optimal matching problem (Table 7 below). Nevertheless, the hyperparameter k can be set to be much smaller than the data dimensionality, whilst in most cases providing a substantial gain in computational performance over the matching on a fully connected graph.

Table 7. Memory usage and computation time of the optimal matching as the $k$ hyperparameter in the kNN search increases. The table indicates the values obtained on the downsampled TuPro dataset, consisting of 4.683 cells per technology.

| k | Max Memory [Mb] | CPU time [s] |
| --- | --- | --- |
| 10 | 349.14 | 3.86 |
| 20 | 349.14 | 4.43 |

(continued)

| k | Max Memory [Mb] | CPU time [s] |
|---|---|---|
| 50 | 349.20 | 5.43 |
| 100 | 347.48 | 6.43 |
| 150 | 347.47 | 7.86 |
| 320 | 414.90 | 4.57 |
| 500 | 538.60 | 4.50 |
| 1000 | 826.68 | 7.57 |
| 1500 | 1119.6 | 9.71 |
| 2000 | 1412.5 | 12.43 |
| 2500 | 1705.3 | 15.43 |
| 3000 | 1998.1 | 17.57 |
| 3500 | 2289.2 | 20.29 |
| 4000 | 2583.2 | 23.00 |
| 4683 | 2983.9 | 26.29 |

Similar results were observed for the effect of varying the hyperparameter k in the application of the Minimum-Cost Maximum-Flow matching algorithm as illustrated in Table 8 below. Whilst the overall speed of this algorithm is slower, the method has other advantages in terms of scalability and providing trade-offs between true and false positive rates.

Table 8. Memory usage and computation time of the MCMF matching algorithm as the $k$ hyperparameter in the kNN search increases. The table indicates the values obtained on the downsampled TuPro dataset, consisting of 4.683 cells per technology.

| k | Max Memory [Mb] | CPU time [s] |
|---|---|---|
| 5 | 1674.9 | 1492.7 |
| 10 | 2456.3 | 2803.3 |
| 25 | 4690.1 | 7723.7 |
| 50 | 8740.3 | 7441.3 |
| 75 | 12344 | 12656 |
| 100 | 17002 | 17331 |
| 125 | 20606 | 22857 |
| 150 | 24213 | 29077 |

*Conclusions*

The method of the present embodiment provides a technology-invariant approach that pairs single-cell measurements across multi-modal datasets, without requiring feature correspondences, making real multi-modal single-cell analysis possible, and opening up new opportunities to gain a multi-view understanding of the dynamics of individual cells in various disease or developmental states. The underlying auto-encoder framework combined with a customized bipartite matching approach ensures scalability.

By introducing a divergence measure, common problems in adversarial training like training instability and convergence problems were addressed.

Scalability was provided by using a modified bipartite matching solution to efficiently match corresponding cells across technologies. The modifications and extensions to the bipartite matching can provide a wider applicability of our method, since shifts in cell-type composition across disjoint aliquots, even coming from the same sample, can be expected. Furthermore, the introduction of the null node ensures a higher quality of matches, by avoiding forced mismatches and thus, improving confidence in the cell-to-cell assignments.

With increasing data dimensionality, the number of nearest neighbours (k) should also rise, since more ties are likely to occur. Nevertheless, the difference in the number of True Positives across various values of k for the same dataset stays within 5% in the experiments carried out and hence it is considered that the performance is quite robust against the choice of this hyperparameter.

While the specific embodiment described above uses two datasets, it will be appreciated that the approaches described can be extended to situations in which there are three (or more) datasets. The latent space may be constructed using all of the datasets simultaneously, with the objective function adapted accordingly. Further, for larger numbers of datasets, it is believed to remain most computationally efficient to perform the matching using a bipartite matching approach between each potential pair of datasets.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. Preferably the computer system has a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Those skilled in the art will appreciate that while the foregoing has described embodiments of the present invention, the present invention should not be limited to the specific configurations and methods disclosed in this description of the preferred embodiments. Those skilled in the art will recognise that present invention has a broad range of applications, and that the embodiments may take a wide range of modifications without departing from any inventive concept as defined in the appended claims.

The following references are hereby incorporated by reference in their entirety:

Abadi, M. et al. (2015). TensorFlow: Large-scale machine learning on heterogeneous systems. Software available from tensorflow.org.

Ahuja, R. K. et al. (1993). Network Flows: Theory, Algorithms, and Applications. Prentice-Hall, Inc., USA.

Amodio, M. and Krishnaswamy, S. (2018). MAGAN: Aligning biological manifolds. In J. Dy and A. Krause, editors, Proceedings of the 35th International Conference on Machine Learning, volume 80 of Proceedings of Machine Learning Research, pages 215-223, Stockholmsmässan, Stockholm Sweden. PMLR.

Bandura, D. R. et al. (2009). Mass cytometry: Technique for real time single cell multitarget immunoassay based on inductively coupled plasma time-of-flight mass spectrometry. Analytical Chemistry, 81(16), 6813-6822.

Chevrier, S. et al. (2017). An immune atlas of clear cell renal cell carcinoma. Cell, 169(4), 736 - 749.e18.

Chevrier, S. et al. (2018). Compensation of signal spillover in suspension and imaging mass cytometry. Cell Systems, 6(5), 612 - 620.e5.

Dell'Amico, M. and Toth, P. (2000). Algorithms and codes for dense assignment problems: the state of the art. Discrete Applied Mathematics, 100(1), 17 - 48.

Giesen, C. et al. (2014). Highly multiplexed imaging of tumor tissues with subcellular resolution by mass cytometry. Nature Methods, 11(4), 417-422.

Heusel, M. et al. (2017). GANs trained by a two Time-Scale update rule converge to a local nash equilibrium.

Irmisch, A. et al. (2020). The tumor profiler study: Integrated, multi-omic, functional tumor profiling for clinical decision support. medRxiv.

Jonker, R. and Volgenant, A. (1987). A shortest augmenting path algorithm for dense and sparse linear assignment problems. Computing, 38(4), 325-340.

Kingma, D. P. and Ba, J. (2014). Adam: A method for stochastic optimization.

Kingma, D. P. and Welling, M. (2013). Auto-Encoding variational bayes. Klein, M. (1967). A primal method for minimal cost flows with applications to the assignment and transportation problems. Management Science, 14(3), 205-220.

Kovács, P. (2015). Minimum-cost flow algorithms: an experimental evaluation. Optimization Methods and Software, 30(1), 94-127.

Liu, J. et al. (2019). Jointly embedding multiple single-cell omics measurements. BioRxiv, page 644310.

Lucic, M. et al. (2017). Are GANs created equal? a Large-Scale study.

Makhzani, A. et al. (2015). Adversarial autoencoders.

Miyato, T. et al. (2018). Spectral normalization for generative adversarial networks.

Oetjen, K. A. et al. (2018). Human bone marrow assessment by single-cell rna sequencing, mass cytometry, and flow cytometry. JCI Insight, 3(23).

Papadopoulos, N. et al. (2019). PROSSTT: probabilistic simulation of single-cell RNA-seq data for complex differentiation processes. Bioinformatics, 35(18), 3517-3519.

Rozenblatt-Rosen, O. et al. (2017). The human cell atlas: from vision to reality. Nature News, 550(7677), 451.

Salimans, T. et al. (2016). Improved techniques for training GANs. In D. D. Lee, M.

Sugiyama, U. V. Luxburg, I. Guyon, and R. Garnett, editors, Advances in Neural Information Processing Systems 29, pages 2234-2242. Curran Associates, Inc.

Stoeckius, M. et al. (2017). Simultaneous epitope and transcriptome measurement in single cells. Nature Methods, 14, 865.

Tang, F. et al. (2009). mrna-seq whole-transcriptome analysis of a single cell. Nature Methods, 6(5), 377-382.

Tirosh, I. et al. (2016). Dissecting the multicellular ecosystem of metastatic melanoma by single-cell rna-seq. Science, 352(6282), 189-196.

Wang, Q. et al. (2009). Divergence estimation for multidimensional densities via k-nearest-neighbor distances. IEEE Transactions on Information Theory, 55(5), 2392-2405.

Wang, T. et al. (2019). Bermuda: a novel deep transfer learning method for single-cell rna sequencing batch correction reveals hidden high-resolution cellular subtypes. Genome Biology, 20(1), 165.

Welch, J. D. et al. (2017). Matcher: manifold alignment reveals correspondence between single cell transcriptome and epigenome dynamics. Genome Biology, 18(1), 138.

Yang, K. D. and Uhler, C. (2019). Multi-domain translation by learning uncoupled autoencoders. CoRR, abs/1902.03515.

Zhu, C. et al. (2020). Single-cell multimodal omics: the power of many.
Nature Methods, 17(1), 11-14.

## Claims

1. A method of determining a correspondence between a first biological property of a cell contained in a first set of biological properties determined by a first analysis technique and a second biological property of a cell contained in a second set of biological properties determined by a second, different, analysis technique, the method including the steps of:

   converting the first and second sets of biological properties into corresponding representations in a representation format which is invariant to the technologies used to derive the biological properties;
   determining, in said representation format, a second representation from the converted second set of biological properties which most closely matches a first representation of the first biological property; and
   re-converting the second representation from the representation format back to the biological property associated with the second representation and thereby determining a correspondence between the first biological property and the second biological property.

2. A method of determining a correspondence between a first biological property of a cell and a plurality of further biological properties of cells, the first biological property and the further biological properties each being determined by different analysis techniques and each being contained in a respective one of a plurality of sets of biological properties, the method including the steps of:

   converting the plurality of sets of biological properties into corresponding representations in a representation format which is invariant to the technologies used to derive the biological properties;
   determining, in said representation format, a representation from each of the converted sets of further biological properties which most closely matches the first representation of the first biological property; and
   re-converting the determined representations from the representation format back to the biological properties associated with the determined representations and thereby determining a correspondence between the first biological property and each of the further biological properties.

3. A method according to claim 1 or claim 2 wherein at least one of the analysis techniques is a single-cell analysis technique.

4. A method according to any one of the preceding claims wherein the representation which is invariant to the technologies used to derive the biological properties is a latent space.

5. A method according to claim 4 wherein the latent space is constructed by creating neural networks for a) an encoder for each data set; b) a decoder for each data set; and c) a discriminator acting on the representations.

6. A method according to claim 5 wherein the discriminator is a binary classifier.

7. A method according to claim 5 or claim 6 wherein the latent space is constructed by minimizing the reconstruction error between the encoder and decoder for each data set while adversarially fooling the discriminator.

8. A method according to any one of the preceding claims wherein the step of determining uses a bipartite matching approach between each pair of the converted sets.

9. A method according to claim 8 wherein the step of determining includes the sub-step of reducing the search space using a k-Nearest Neighbour approach to reduce the number of possible correspondences to a pre-determined maximum.

10. A method according to claim 8 or claim 9 wherein the bipartite matching approach uses a minimum-cost maximum-flow algorithm.

11. A method according to any one of the preceding claims wherein the step of determining allows for the possibility of a correspondence not being found by adding a null node to each converted set.

12. A method according to any one of the preceding claims wherein the step of determining allows for many-to-one matches to representations in a converted set which has fewer elements.

13. A computer program arranged, when run on a processor, to perform the method of any one of the above claims.

14. A computer program product having non-transitory memory storing the computer program of claim 13.

15. A computer system having a processor which is arranged to perform the method of any one of claims 1 to 12.

1

Fig. 1

Fig. 2

Fig. 3

Fig. 6

Fig. 4

Fig. 5

Fig. 7

Fig. 8

Fig. 9

Comparison of matching accuracy
using sparse and dense connections

Fig. 11

Fig. 10

Fig. 12

Fig. 13

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WELCH J D ET AL: "MATCHER: manifold alignment reveals correspondence between single cell transcriptome and epigenome dynamics", GENOME BIOLOGY, vol. 18, no. 1, 24 July 2017 (2017-07-24), XP055675674, DOI: 10.1186/s13059-017-1269-0 | 1-4, 13-15 | INV. G16B40/30 G16H50/20 C12Q1/68 |
| Y | * abstract; figure 1 * * p.2 col.1 par.2, p.3 col.1 par.1, 3, p.4 col.1 par.1, 3; figure 1e * ----- | 5-12 | |
| X | WO 2020/068731 A1 (MASSACHUSETTS GEN HOSPITAL [US]; BROAD INST INC [US] ET AL.) 2 April 2020 (2020-04-02) | 1-4, 13-15 | |
| Y | * p.2 par.3, p.4 par.3-4,p.8 par.3-4, par. across pp.11-12, p.12 par.4-p.14 par.1; figures 1, 3, 5-6 * ----- | 5-12 | |
| X | WELCH J D ET AL: "Single-Cell Multi-omic Integration Compares and Contrasts Features of Brain Cell Identity", CELL, vol. 177, no. 7, 13 June 2019 (2019-06-13), page 1873, XP085712715, ISSN: 0092-8674, DOI: 10.1016/J.CELL.2019.05.006 | 1-4, 13-15 | **TECHNICAL FIELDS SEARCHED (IPC)** G16B G16H C12Q |
| Y | * abstract; figure 1 * * p.1881 col.1 par.3-col.2 par.3; figure 5 * * p.1881 col.2 par.4-p.1885 col.1 par.1; figures 6-7 * * "Methods" * ----- -/-- | 5-12 | |

The present search report has been drawn up for all claims

| Place of search | Date of completion of the search | Examiner |
|---|---|---|
| Munich | 11 September 2020 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/020419 A1 (KAHVEJIAN AVAK [US] ET AL) 16 January 2020 (2020-01-16) | 1-4, 13-15 | |
| Y | * [0012]-[0014], [0026], [0030]-[0031], [0164]-[0165]; figure 3 * | 5-12 | |
| | ----- | | |
| X | STUART T ET AL: "Comprehensive Integration of Single-Cell Data", CELL, vol. 177, no. 7, 13 June 2019 (2019-06-13), page 1888, XP085712713, ISSN: 0092-8674, DOI: 10.1016/J.CELL.2019.05.031 | 1-4, 13-15 | |
| Y | * abstract; figure 1 * | 5-12 | |
| | ----- | | |
| Y,D | AMODIO M ET AL: "MAGAN: Aligning Biological Manifolds", ARXIV.ORG, CORNELL UNIVERSITY LIBRARY, 201 OLIN LIBRARY CORNELL UNIVERSITY ITHACA, NY 14853, 10 February 2018 (2018-02-10), pages 1-8, XP081213924, * abstract; figure 1 * * p.1889 col.1 par.2-col.2 par.2 * | 5-12 | TECHNICAL FIELDS SEARCHED (IPC) |
| | ----- | | |
| Y,D | YANG K D ET AL: "Multi-Domain Translation by Learning Uncoupled Autoencoders", ARXIV.ORG, CORNELL UNIVERSITY LIBRARY, 201 OLIN LIBRARY CORNELL UNIVERSITY ITHACA, NY 14853, 10 February 2019 (2019-02-10), XP081027312, * p.2 col.1 par.2, p.8 col.2 par.1; figure 7 * | 5-12 | |
| | ----- | | |
| | -/-- | | |

The present search report has been drawn up for all claims

| Place of search | Date of completion of the search | Examiner |
|---|---|---|
| Munich | 11 September 2020 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LOTFOLLAHI M ET AL: "scGen predicts single-cell perturbation responses", NATURE METHODS, NATURE PUB. GROUP, NEW YORK, vol. 16, no. 8, 29 July 2019 (2019-07-29), pages 715-721, XP036847272, ISSN: 1548-7091, DOI: 10.1038/S41592-019-0494-8 [retrieved on 2019-07-29] * p.718, "cross-study prediction"; figure 4 * * "Methods" * ----- | 1-15 | |
| A | WO 2020/077352 A1 (HUMAN LONGEVITY INC [US]) 16 April 2020 (2020-04-16) * [0094]-[0096], [0130], [0165]; claims 1, 16-21 * ----- | 1-15 | |
| A | WO 2019/018693 A2 (ALTIUS INST FOR BIOMEDICAL SCIENCES [US]) 24 January 2019 (2019-01-24) * [0003]-[0007], [00110], [00124], [00132]; claims 1, 6-13 * ----- | 1-15 | **TECHNICAL FIELDS SEARCHED (IPC)** |

The present search report has been drawn up for all claims

| Place of search | Date of completion of the search | Examiner |
|---|---|---|
| Munich | 11 September 2020 | Werner, Andreas |

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2020

| Patent document cited in search report | | | Publication date | Patent family member(s) | | | Publication date |
|---|---|---|---|---|---|---|---|
| WO 2020068731 | A1 | | 02-04-2020 | NONE | | | |
| US 2020020419 | A1 | | 16-01-2020 | US | 2020020419 | A1 | 16-01-2020 |
| | | | | WO | 2020018519 | A1 | 23-01-2020 |
| WO 2020077352 | A1 | | 16-04-2020 | NONE | | | |
| WO 2019018693 | A2 | | 24-01-2019 | US | 2020167914 | A1 | 28-05-2020 |
| | | | | WO | 2019018693 | A2 | 24-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AHUJA, R. K. et al.** Network Flows: Theory, Algorithms, and Applications. Prentice-Hall, Inc, 1993
- **AMODIO, M. ; KRISHNASWAMY, S.** MAGAN: Aligning biological manifolds. *Proceedings of the 35th International Conference on Machine Learning, volume 80 of Proceedings of Machine Learning Research,* 2018, 215-223
- **BANDURA, D. R.** Mass cytometry: Technique for real time single cell multitarget immunoassay based on inductively coupled plasma time-of-flight mass spectrometry. *Analytical Chemistry,* 2009, vol. 81 (16), 6813-6822
- **CHEVRIER, S. et al.** An immune atlas of clear cell renal cell carcinoma. *Cell,* 2017, vol. 169 (4), 736-749
- **CHEVRIER, S. et al.** Compensation of signal spillover in suspension and imaging mass cytometry. *Cell Systems,* 2018, vol. 6 (5), 612-620
- **DELL'AMICO, M. ; TOTH, P.** Algorithms and codes for dense assignment problems: the state of the art. *Discrete Applied Mathematics,* 2000, vol. 100 (1), 17-48
- **GIESEN, C. et al.** Highly multiplexed imaging of tumor tissues with subcellular resolution by mass cytometry. *Nature Methods,* 2014, vol. 11 (4), 417-422
- **HEUSEL, M. et al.** *GANs trained by a two Time-Scale update rule converge to a local nash equilibrium,* 2017
- **IRMISCH, A. et al.** The tumor profiler study: Integrated, multi-omic, functional tumor profiling for clinical decision support. *medRxiv,* 2020
- **JONKER, R. ; VOLGENANT, A.** A shortest augmenting path algorithm for dense and sparse linear assignment problems. *Computing,* 1987, vol. 38 (4), 325-340
- **KINGMA, D. P. ; BA, J.** *Adam: A method for stochastic optimization,* 2014
- **KINGMA, D. P. ; WELLING, M.** *Auto-Encoding variational bayes,* 2013
- **KLEIN, M.** A primal method for minimal cost flows with applications to the assignment and transportation problems. *Management Science,* 1967, vol. 14 (3), 205-220
- **KOVÁCS, P.** Minimum-cost flow algorithms: an experimental evaluation. *Optimization Methods and Software,* 2015, vol. 30 (1), 94-127
- **LIU, J. et al.** Jointly embedding multiple single-cell omics measurements. *BioRxiv,* 2019, 644310
- **LUCIC, M. et al.** *Are GANs created equal? a Large-Scale study,* 2017
- **MAKHZANI, A. et al.** *Adversarial autoencoders,* 2015
- **MIYATO, T. et al.** *Spectral normalization for generative adversarial networks,* 2018
- **OETJEN, K. A. et al.** Human bone marrow assessment by single-cell rna sequencing, mass cytometry, and flow cytometry. *JCI Insight,* 2018, vol. 3 (23
- **PAPADOPOULOS, N. et al.** PROSSTT: probabilistic simulation of single-cell RNA-seq data for complex differentiation processes. *Bioinformatics,* 2019, vol. 35 (18), 3517-3519
- **ROZENBLATT-ROSEN, O. et al.** The human cell atlas: from vision to reality. *Nature News,* 2017, vol. 550 (7677), 451
- Improved techniques for training GANs. **SALIMANS, T.** Advances in Neural Information Processing Systems. Curran Associates, Inc, 2016, vol. 29, 2234-2242
- **STOECKIUS, M. et al.** Simultaneous epitope and transcriptome measurement in single cells. *Nature Methods,* 2017, vol. 14, 865
- **TANG, F. et al.** mrna-seq whole-transcriptome analysis of a single cell. *Nature Methods,* 2009, vol. 6 (5), 377-382
- **TIROSH, I. et al.** Dissecting the multicellular ecosystem of metastatic melanoma by single-cell rna-seq. *Science,* 2016, vol. 352 (6282), 189-196
- **WANG, Q. et al.** Divergence estimation for multidimensional densities via k-nearest-neighbor distances. *IEEE Transactions on Information Theory,* 2009, vol. 55 (5), 2392-2405
- **WANG, T. et al.** Bermuda: a novel deep transfer learning method for single-cell rna sequencing batch correction reveals hidden high-resolution cellular subtypes. *Genome Biology,* 2019, vol. 20 (1), 165
- **WELCH, J. D. et al.** Matcher: manifold alignment reveals correspondence between single cell transcriptome and epigenome dynamics. *Genome Biology,* 2017, vol. 18 (1), 138
- **YANG, K. D. ; UHLER, C.** Multi-domain translation by learning uncoupled autoencoders. *CoRR,* 2019

- **ZHU, C. et al.** *Single-cell multimodal omics: the power of many,* 2020

- *Nature Methods,* vol. 17 (1), 11-14